**Europäisches Patentamt**

**European Patent Office**

**Office européen des brevets**

(11) Veröffentlichungsnummer: **0 066 008**
**B1**

## (12) EUROPÄISCHE PATENTSCHRIFT

(45) Veröffentlichungstag der Patentschrift:
**16.01.85**

(51) Int. Cl.⁴: **A 61 B 6/00,** H 05 G 1/46

(21) Anmeldenummer: **81108429.2**

(22) Anmeldetag: **16.10.81**

(54) **Röntgendiagnostikanlage für angiographische Röntgenuntersuchungen.**

(30) Priorität: **03.06.81 DE 3122098**

(43) Veröffentlichungstag der Anmeldung:
**08.12.82 Patentblatt 82/49**

(45) Bekanntmachung des Hinweises auf die Patenterteilung:
**16.01.85 Patentblatt 85/3**

(84) Benannte Vertragsstaaten:
**DE FR GB**

(56) Entgegenhaltungen:
**DE - A - 1 921 592**
**DE - A - 2 647 928**
**FR - A - 2 406 187**

**ELECTROMEDICA, Band 45, Nr. 3/4, 1977, Seiten 133-136, Erlangen, DE, G. FREDZELL et al.: "Koordinat Angio - eine Basiseinheit für den Spezialuntersuchungsraum"**

(73) Patentinhaber: **Siemens Aktiengesellschaft, Berlin und München Wittelsbacherplatz 2, D-8000 München 2 (DE)**

(72) Erfinder: **Pfeifer, Rolf, Breslauer Strasse 9, D-8520 Erlangen (DE)**

# Beschreibung

Die Erfindung betrifft eine Röntgendiagnostikanlage gemäss dem Oberbegriff des Patentanspruches.

Eine Röntgendiagnostikanlage dieser Art ist beispielsweise im IEEE-Katalog, Nr. 76CH1160-1C, 1976, S. 255 bis 260, beschrieben. Dabei ist es möglich, im Bildspeicher ein Röntgenbild oder mehrere auf integrierte Röntgenbilder zu speichern, die einem Leerbild des Aufnahmeobjektes entsprechen, und die Differenz aus den gespeicherten Bildinformationen und Bildinformationen zu bilden, die demselben Bereich des Aufnahmeobjektes entsprechen, wobei jedoch in die Blutgefässe ein Röntgenkontrastmittel eingeführt ist. Auf diese Weise kann in Echtzeit ein Röntgendurchleuchtungsbild erhalten werden, das nur die für die Diagnose wichtigen Blutgefässe, die mit Kontratsmittel gefüllt sind, zeigt. Es ist auch möglich, die zu untersuchende Bildszene mit Hilfe eines Videorecorders aufzunehmen und die Bildsubtraktion anhand der aufgezeichneten Szene vorzunehmen.

Durch die Zeitschrift „Electromedica", 45, Nr. 3/4, 1974, S. 133 bis 136, ist eine Röntgendiagnostikanlage für angiographische Röntgenuntersuchungen bekannt, die dem Personal die Bedienung erleichtert und servicefreundlich ist. Diese bekannte Röntgendiagnostikanlage erlaubt aber nicht die Wiedergabe von Subtraktionsbildern und befasst sich demgemäss auch nicht mit der Bedienungsvereinfachung für die Erzeugung solcher Bilder.

Durch die DE-A Nr. 2647928 ist ebenfalls eine Röntgendiagnostikanlage für angiographische Röntgenuntersuchungen bekannt, die die Wiedergabe von Subtraktionsbildern nicht erlaubt. Bei dieser Röntgendiagnostikanlage sind mehrere beleuchtbare Tasten vorgesehen, die entsprechend dem jeweiligen Untersuchungsablauf beleuchtet werden. Diese Tasten tragen Symbole für den jeweiligen Untersuchungsschritt und sind unmittelbar nebeneinander in einem Tastenfeld angeordnet, so dass die Anzeige relativ unübersichtlich ist.

Der Erfindung liegt die Aufgabe zugrunde, eine Röntgendiagnostikanlage gemäss dem Oberbegriff des Patentanspruches so auszubilden, dass eine einfache und übersichtliche Bedienung und Überwachung des Untersuchungsablaufes möglich sind.

Diese Aufgabe ist erfindungsgemäss durch die im Kennzeichen des Patentanspruches angegebene Ausbildung gelöst. Bei der erfindungsgemässen Röntgendiagnostikanlage ist am Bedienpult die jeweils erreichte Untersuchungsstufe eines Gesamtuntersuchungsablaufes in der Darstellung für den zeitlichen Kontrastmittelverlauf optisch erkennbar, so dass beispielsweise in einfacher Weise überprüfbar ist, welche Stufe eines gesamten Untersuchungsvorganges jeweils erreicht ist und die folgende Stufe eingeleitet werden kann. So ist es beispielsweise leicht möglich, nach Anzeige der Einschaltung der Durchleuchtung durch Tastendruck eine Kontrastmittelinjektion auszulösen, anschliessend die Einspeicherung eines Leerbildes zu bewirken, dann die Einspeicherung eines Füllungsbildes (Blutgefässe mit Kontrastmittel gefüllt) in einen weiteren Bildspeicher auszulösen und schliesslich den Untersuchungsablauf zu beenden. Das Füllungsbild wird vom Leerbild elektronisch subtrahiert und das Differenzbild auf einem Monitor wiedergegeben.

Die Erfindung ist nachfolgend anhand eines in der Zeichnung dargestellten Ausführungsbeispieles näher erläutert. Es zeigen:

Fig. 1 eine Blockdarstellung einer Röntgendiagnostikanlage nach der Erfindung, und

Fig. 2 die Bedienfläche des Bedienpultes der Röntgendiagnotikanlage gemäss Fig. 1.

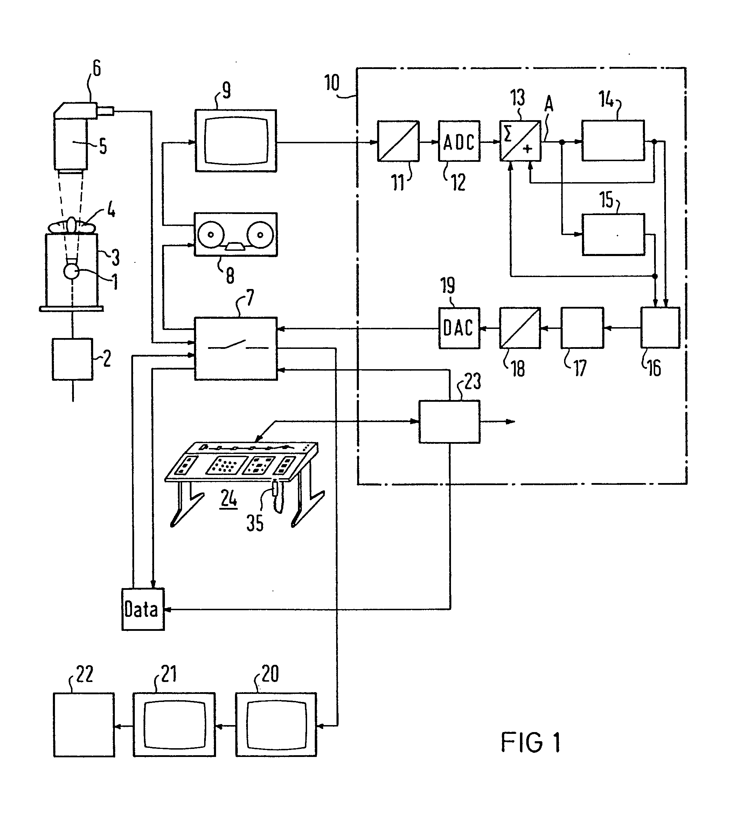

In der Fig. 1 ist eine Röntgenröhre 1 dargestellt, die von einem Röntgengenerator 2 gespeist wird und einen auf einem Röntgentisch 3 liegenden Patienten 4 durchstrahlt. Eine mit einem Röntgenbildverstärker 5 und einer Fernsehkamera 6 bestückte Bildverstärker-Fernsehkette nimmt die Röntgenbilder auf und setzt sie in ein Videosignal um, das einem Videoverteiler 7 zugeführt wird. Der Videoverteiler 7 steuert die Aufzeichnung der Röntgenbilder auf einem Videorecorder 8 und die Wiedergabe dieser Bilder auf einem Monitor 9.

Zur Erzeugung von Differenzbildern wird das Videosignal entweder in Echtzeit oder das vom Videorecorder 8 gelieferte Videosignal einer Bildsubtraktionsvorrichtung 10 zugeführt, an deren Eingang ein Logarithmierer 11 liegt, dessen Ausgangssignal einem Analog-Digital-Wandler 12 zugeführt wird. Der Logarithmierer 11 bewirkt, dass Signale voneinander subtrahiert werden, die proportional der Summe der Produkte aus Massenschwächungskoeffizient und Masse aller Stoffe, die im Strahlengang liegen, sind. Dem Analog-Digital-Wandler 12 ist ein Mittelwertbildner 13 nachgeschaltet, der eine gleitende, gewichtete Mittelwertbildung zur Störabstandsverbesserung vornimmt. Es kann auch eine Summation von Bildsignalen erfolgen. Die so gewonnenen Bildsignale werden in zwei Bildspeichern 14 und 15 eingespeichert, die an einem Subtrahierer 16 angeschlossen sind, dessen Ausgangssignal über einen Fensterverstärker 17, einen Delogarithmierer 18 und einen Digital-Analog-Wandler 19 dem Videoverteiler 7 zugeführt wird, der die Wiedergabe der Subtraktionsbilder auf Monitoren 20 und 21 bewirkt. Die Subtraktionsbilder können mit Hilfe einer Bildaufnahmeeinrichtung 22 dauerhaft festgehalten, z.B. photographiert werden.

Für die Herstellung von angiographischen Subtraktionsbildern wird nach Injektion eines Kontrastmittels in ein Blutgefäss, noch bevor sich das Kontrastmittel in dem zu untersuchenden Gefässbereich ausgebreitet hat, im Bildspeicher 14 eine Maske gesetzt, die mehreren Leerbildern entspricht. Die gemittelten bzw. integrierten Leerbilder werden im Bildspeicher 14 gespeichert. Anschliessend wird möglichst bei der maximalen Kontrastmittelfüllung des zu untersuchenden Gefässbereiches eine Mittelung bzw. Integration einiger Fernsehbilder bewirkt und diese Füllungsbilder werden im Bildspeicher 15 gespeichert. Werden die Inhalte der Speicher 14 und 15 durch den Subtrahierer 16 voneinander subtrahiert, so können auf den Monitoren 20 und 21 Subtraktionsbilder wiedergegeben werden, die nur die mit Kontrastmittel gefüllten Gefässe zeigen, den Untergrund, der immer gleich ist, jedoch nicht mehr enthalten.

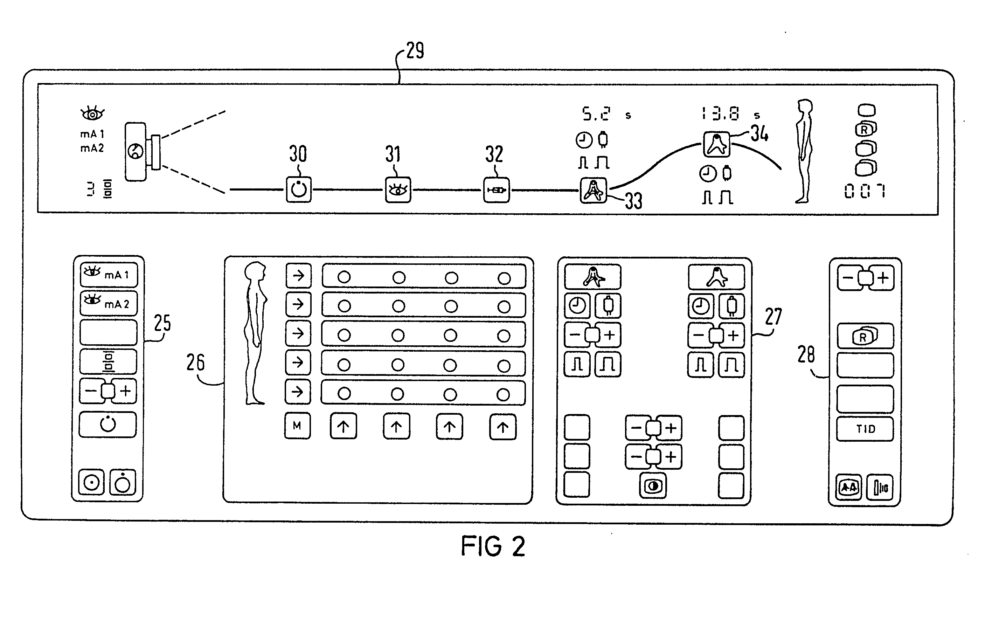

Zur Steuerung der gesamten Röntgendiagnostikanlage ist eine Steuervorrichtung 23 vorhanden, die von einem Bedienpult 24 angesteuert wird. Das Bedienpult 24 ist in der Fig. 2 näher dargestellt.

Aus der Fig. 2 geht hervor, dass die Bedienfläche des Bedienpultes 24 vier Bereiche 25 bis 28 aufweist, in denen Tasten angeordnet sind. Im Bereich 25 sind dabei Tasten angeordnet, die der Wahl der Aufnahmewerte dienen, im Bereich 27 Tasten, die der Steuerung des Untersuchungsablaufes dienen, im Bereich 28 Tasten, die der Steuerung der Bildaufzeichnung dienen, und im Bereich 26 sind Tasten angeordnet, die es ermöglichen, bestimmte, in den Bereichen 25, 27, 28 gewählte Werte dauerhaft festzuhalten, also abhängig von Körperregion (Organ) und Untersuchungsart zu programmieren. Über den Bereichen 25 bis 28 ist ein Anzeigefeld 29 mit einer schematischen Darstellung des Kontrastmittelverlaufes in einem Blutgefäss angeordnet. In dem Anzeigefeld 29, und zwar in dem Kontrastmittelverlauf, sind Anzeigemittel 30 bis 34 für die einzelnen Stufen des Untersuchungsablaufes vorgesehen, die an der Steuervorrichtung 23, die auch den Röntgengenerator 2 steuert, angeschlossen sind und von ihr derart gesteuert werden, dass sie die jeweilige Untersuchungsstufe anzeigen. Jedes der Anzeigemittel 30 bis 34 liegt dabei an der ihm im Kontrastmittelverlauf zugeordneten Stelle. Demgemäss werden die Anzeigemittel 30 bis 34 nacheinander eingeschaltet. Durch das Anzeigemittel 30 wird die Vorbereitung der Röntgendiagnostikanlage für die Erstellung eines Subtraktionsbildes angezeigt, durch das Anzeigemittel 31 die Einschaltung der Durchleuchtung, durch das Anzeigemittel 32 die Kontrastmittelinjektion, durch das Anzeigemittel 33 die Einspeicherung eines Leerbildes in den Bildspeicher 14 (Maske) und durch das Anzeigemittel 34 die Einspeicherung eines Füllungsbildes in den Bildspeicher 15. Diese Einspeicherung wird dabei mit Hilfe von Tasten im Bereich 27 angewählt und erfolgt durch Betätigung eines Auslösers 35 (Fig. 1) am Bedienpult 24.

Die Fig. 2 zeigt, dass das Bedienpult alle für die Einstellung und Steuerung der Röntgendiagnostikanlage erforderlichen Bedienelemente in übersichtlicher Weise vereinigt, so dass eine besonders einfache und übersichtliche Bedienung möglich ist.

Bei dem in der Fig. 1 dargestellten Ausführungsbeispiel für eine Röntgendiagnostikanlage für angiographische Röntgenuntersuchungen sind zwei Bildspeicher 14 und 15 vorgesehen. Im Bildspeicher 15 wird dabei ein Bild gespeichert, das zu einem anderen Zeitpunkt als das Bild anfällt, das im Bildspeicher 14 als Maske gespeichert ist. Es ist auch möglich, auf den Bildspeicher 15 zu verzichten, wenn das jeweilige aktuelle Videosignal von der im Bildspeicher 14 gespeicherten Bildinformation subtrahiert wird.

## Patentanspruch

Röntgendiagnostikanlage für angiographische Röntgenuntersuchungen mit einer Bildverstärker-Fernsehkette (5, 6, 9) und einer daran angeschlossenen Bildsubtraktionsvorrichtung (10) mit mindestens einem Bildspeicher (14), dessen Bildinformationen von Bildinformationen subtrahiert werden, die zu anderen Zeitpunkten als die gespeicherten Bildinformationen anfallen, und mit einer Wiedergabevorrichtung (20, 21) für die Subtraktionsbilder, bei der eine Steuervorrichtung (23) für die Bildverarbeitung vorhanden ist, dadurch gekennzeichnet, dass ein zentrales Bedienpult (24) vorhanden ist, das eine schematische Darstellung eines typischen zeitlichen Verlaufes der Kontrastmittelkonzentration in einem Blutgefäss bei derartigen angiographischen Röntgenuntersuchungen zeigt, in dem Anzeigemittel (30 bis 34) für einzelne Stufen des Untersuchungsablaufes an den jeweils zugeordneten Stellen liegen, die an der Steuervorrichtung (23) angeschlossen sind und von ihr derart gesteuert werden, dass sie die jeweilige Untersuchungsstufe anzeigen.

## Claim

X-ray diagnostic apparatus for angiographic X-ray examinations having an image intensifier television chain (5, 6, 9) connected to an image subtraction device (10) having at least one image store (14) in which is stored information to be subtracted from image information obtained subsequent to the obtaining of the stored image information, and having a reproducing device (20, 21) for the subtraction images, with a control device (23) for image processing, characterised in that a central operating panel (24) is provided which illustrates a schematic illustration of a typical time course of the medium contrast concentration in a blood vessel in such angiographic X-ray examinations, in which operating panel are arranged indicating means (30 to 34) for the individual stages of the examination sequence at the respectively assigned places connected to the control device (23) and controlled thereby in such manner that they indicate the respective examination stage.

## Revendication

Installation de radiodiagnostic pour des examens angiographiques comportant une chaîne de télévision à amplificateur de brillance (5, 6, 9) et un dispositif de soustraction d'images (10) qui est raccordé à cette chaîne de télévision et comporte au moins une mémoire d'images (14), dont les informations d'images sont soustraites d'informations d'images qui arrivent à d'autres instants que les informations mémorisées, et comportant un dispositif (20, 21) de reproduction des images de soustraction, et dans lequel il est prévu un dispositif de commande (23) pour le traitement des images, caractérisée par le fait qu'il est prévu un pupitre de commande central (24) qui fournit une représentation schématique d'une variation typique dans le temps d'une concentration de l'agent opacifiant dans un vaisseau sanguin dans le cas de tels examens angiographiques, et dans lequel sont situés des dispositifs d'affichage (30 à 34) pour différentes phases du déroulement de l'examen, en des emplacements associés qui sont raccordés au dispositif de commande (23) et sont commandés par ce dernier de telle sorte qu'ils affichent la phase respective de l'examen.

FIG 1

FIG 2